# EUROPEAN PATENT SPECIFICATION

(11) **EP 1 395 199 B1**
(45) Date of publication and mention of the grant of the patent: **04.05.2005**
(21) Application number: 01998280.0
(22) Date of filing: 28.11.2001
(51) Int. Cl.: A61F 2/00

(54) **MEDICAL DEVICE**
MEDIZINISCHE VORRICHTUNG
DISPOSITIF MEDICAL

(30) Priority: 28.11.2000 GB 0029015
(43) Date of publication of application: 10.03.2004
(73) Proprietor: UNIVERSITY COLLEGE LONDON, London WC1E 6BT (GB)
(72) Inventor: HOLLINGSWORTH, Simon John, Department of Surgery, London W1N 8AA (GB); BARKER, Stephen George E, Department of Surgery, London W1N 8AA (GB)
(74) Representative: Woods, Geoffrey Corlett
(86) International application number: PCT/GB2001/005252
(87) International publication number: WO 2002/043614

(56) References cited:
- T.S. BHATTI ET AL: "Causes of re-recurrence after polytetrafluoroethylene patch saphenoplasty for recurrent varicose veins" BRITISH JOURNAL OF SURGERY, vol. 87, 1 October 2000 (2000-10-01), pages 1356-1360, XP002199847
- G. M. GLASS: "Prevention of Sapheno-femoral and Sapheno-popliteal Recurrence of Varicose Veins by Forming a Partition to Contain Neovascularisation" PHLEBOLOGY, vol. 13, 1998, pages 3-9, XP008002751

## Description

The present invention concerns a device for use in the prevention of recurrent varicose veins.

The occurrence of primary varicose veins is extremely prevalent, for example from national surveys in both Europe and North America, the condition affects approximately 2% of the total population, but incidence increases with age, such that perhaps 30% or more of the over 60's have some degree of varicosity.

Consequently surgery for primary Varicose veins is one of the most common surgical procedures undertaken. For example, in the United Kingdom, in excess of 100,000 operations are performed each year.

Primary varicose vein surgery typically has a successful outcome with relief of the patient's symptoms (especially pain, or aching, often felt following prolonged standing), an acceptable cosmetic result, and a low complication rate (for example wound infection or haematoma formation). However, the recurrence rate of varicose veins is in the region of 10%, and recurrence rates higher than 20% have been reported. Therefore, in the United Kingdom, the number of persons requiring more complex, higher risk, repeat surgery can be in excess of 10,000 per year. Surgery for recurrent varicose veins places a far greater burden on medical resources than does that for primary varicose veins because pre-operative duplex ultrasound imaging becomes necessary, operating times are usually greater and a more prolonged hospital stay is often required. Litigation from "failed" primary varicose vein surgery is also a factor.

Although the causes and prevention of recurrence have been investigated, no satisfactory alleviation of recurrence has been found.

In the article :
"Prevention of Sapheno-femoral and Sapheno-popliteal Recurrence of Varicose Veins"
by dr. G. M. Glass (Phlebology volume 13 , 1998 pages 3 - 9) it is suggested to insert a single artificial patch of silicone near the opening in the cribriform fascia.

It is an object of the present invention to alleviate, at least partially, the above problems.

Accordingly, the present invention provides a medical device comprising first and second lamellar portions joined by a connecting bridge, wherein said device is constructed such that said first and second portions are capable of being positioned on opposite sides of the cribriform fascia, with said connecting bridge passing therethrough, and wherein said first and second portions are barriers to neo-vascularisation.

The present invention can be used in a method of treatment comprising implanting the device according to the invention in a patient.

Preferably the device is implanted to substantially avoid recurrent varicose veins. Preferably the first lamellar portion is positioned to cover potential entry sites of tributaries to the deep femoral vein, sited deep to the cribriform fascia and in particular, the deep pudendal vein. Preferably the second lamellar portion is positioned to prevent substantially neo-vascularisation from superficial long saphenous vein tributaries, sited superficial to the cribriform fascia.

Preferably the method wherein the invention may be used comprises implanting the device at the time of primary varicose vein surgery. This reduces the number of surgical procedures and avoids the more complex, high risk and more resource-consuming surgery for recurrent varicose veins.

Preferably the device is soft and flexible to minimise or eliminate any deep femoral vein or arterial or nerve compression, and to minimise or eliminate any risk of erosion into surrounding structures. Furthermore, it should not be felt transcutaneously. Optionally the device can be sutured into place and optionally the device may be coated with one or more agents, themselves perhaps embedded within a biodegradable matrix such as collagen or gelatin, to deter neo-vascularisation, such as an inhibitor of vascular endothelial growth factor.

Embodiments of the invention will now be described, by way of example only, with reference to the accompanying drawings, in which:
Fig. 1A shows one example of the venous anatomy in the region of the sapheno-femoral junction (known to be extremely variable in this region);
Fig. 1B is a longitudinal cross-section of the junction of Figure 1A;
Fig. 2A is a perspective view of a device embodying the present invention; and
Fig. 2B is a transverse cross-section of the device of Fig. 2A after implantation.

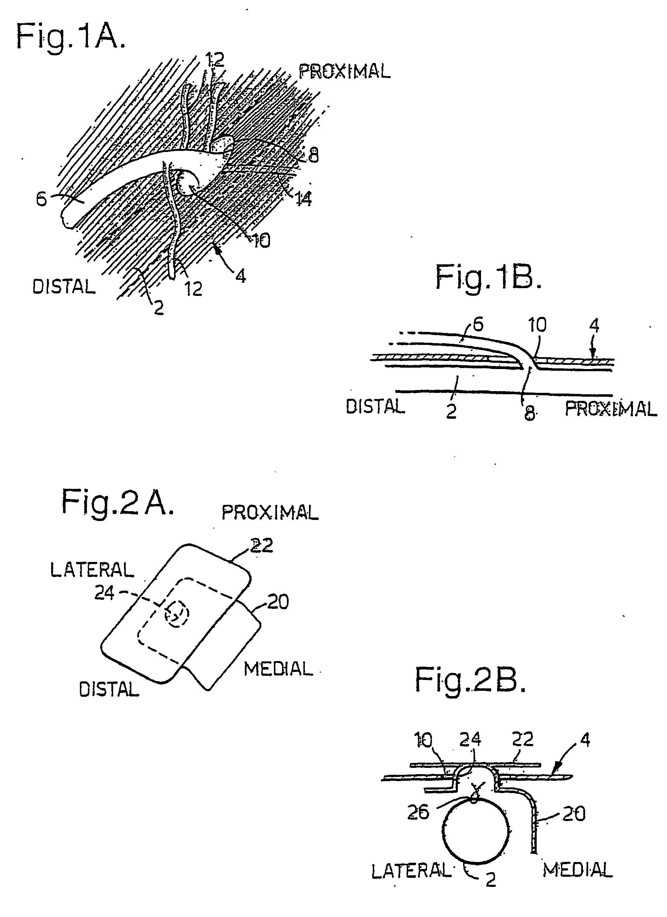

Referring to Figures 1A and 1B, the human anatomy prior to varicose vein surgery will be described to assist understanding of the present invention, although again, it is (known and) emphasised that venous anatomy in this region can be very variable.

The deep femoral vein 2 lies beneath a deep. fascia 4, i.e. a distinct sheet of fibrous tissue. In Fig. 1A a portion of the fascia 4 is shown hatched. In the region of the groin, the long saphenous vein 6 joins the deep femoral vein 2 at the sapheno-femoral junction 8, and in the region of this junction 8, the fascia is far more loose and is known as the cribriform fascia 10. Above the cribriform fascia 10 a plurality of long saphenous vein tributaries 12 join the long saphenous vein 6. Below the cribriform fascia 10, one (or more) deep (pudendal) tributary(ies) 14 commonly join the deep femoral vein 2.

A device embodying the invention is shown in Figure 2A, It consists of a lower part 20 comprising a first lamellar portion and an upper part 22 comprising a second lamellar portion. The lower and upper parts 20, 22 are joined by a connecting bridge 24. The lower and upper parts 20, 22 are approximately rectangular in shape (with rounded corners), although other shapes, for example ovoid, can also be used. The lower part is curved to form a sheet which bends through approximately 90° to conform to the edge of the deep femoral vein. The upper part 22 is oriented such that its long sides lie approximately parallel to the axis of curvature of the lower part 22 and also parallel to the short sides of the lower part 20. The connecting bridge is approximately cylindrical in shape and is formed by a cup-shaped protuberance sculptured from, or additional to, the lower part 20 which connects to the upper part 22.

The device is manufactured from thin, flexible PTFE or Dacron™, or a mesh-type product such as polypropylene or Marlex™. The mesh-type material may be akin to that used for hernia repair. The device is implantable so is either produced sterile or is sterilisable. The device is produced in a range of sizes to suit implantation in patients of different physical size. For example, a range of three sizes, small, medium and large, might typically be produced. Each device is typically a few centimetres long, for example in the range of from 2 to 5 cm, and a few centimetres wide, such as 2 to 3 cm. The thickness of the material of the device is preferably in the range of 0.05 to 0.1 centimetres, but is not limited to that range.

The use of the device in primary varicose vein surgery will now be described with reference to the preferred surgical method for use of the present invention. The sapheno-femoral junction is dissected out. All visible long saphenous vein tributaries 12 above the cribriform fascia 10 are first ligated and then divided. This is followed by a formal dissection below the cribriform fascia 10, exposing the anterior surface of the deep femoral vein 2 both distally and proximally, with subsequent exposure of both its lateral and medial aspects. In particular, any deep pudendal tributary(ies) are exposed, ligated and divided. When satisfied of the relation of the long saphenous vein and the deep femoral vein, the long saphenous vein may be ligated and divided also, leaving a small stump of long saphenous vein at the junction with the deep femoral vein.

In some forms of varicose vein surgery the long saphenous vein is only ligated, but in the preferred method the ligation is followed by stripping of the long saphenous vein 6 and preferably it is stripped at least to knee level.

The device as shown in Figure 2A can then be implanted such that the lower part 20 is placed beneath the cribriform fascia 10 and the upper part 22 above the cribriform fascia 10, as shown in Figure 2B. The device is orientated such that the upper part 22 lies along the line of the deep femoral vein. The portion of the lower part 20, which is curved to form a flap, is implanted deeper in the region of the medial aspect of the deep femoral vein 2. The lower part 20 is implanted such that the axis of curvature is approximately parallel to the deep femoral vein 2, so that the lower part 20 surrounds the periphery of the deep femoral vein, especially on its medial aspect, as shown in Figure 2B. As can also be seen in Figure 2B, the connecting bridge 24 is positioned in the vicinity of over the ligated sapheno-femoral junction 26. The connecting bridge may be approximately 0.5 to 1 centimetres long to allow the tied-off long saphenous vein stump to fit into it.

The shape of the device is such that it holds itself in place due to the surrounding anatomy. However, the device can, optionally, be held in place by use of (absorbable) sutures, until incorporated. Although the device is implanted sterile, per-operative antibiotics can, in addition, be used if necessary.

The composition and thickness of the device mean that it is sufficiently soft and flexible to minimise or eliminate any deep femoral vein or arterial or nerve compression, and to minimise or eliminate any risk of erosion into surrounding structures. Additionally, it cannot be felt transcutaneously.

The device operates as a barrier to prevent neo-vascularisation, and hence recurrence of varicose veins. The upper part 22 prevents neo-vascularisation from any of the more superficial saphenous long vein tributaries 12. The lower part 20, and in particular its medial flap portion, covers the usual potential entry sites of the deep pudendal vein tributary or other tributaries 14. Embodiments of the device constructed of mesh have very small pore size such that small blood vessels are substantially prevented from growing through it Typically the pore size would be the smallest available from manufactures of the mesh material. Recurrences of varicosity are insignificant if they are fed from vessels less than about 1 mm in diameter, even if such vessels managed to grow through the mesh pores at all.

The preferred embodiment of the device has no coating. However, according to a further embodiment of the invention, the device may be used in conjunction with or coated with one or more modulators of an agent which regulates neo-vascularisation, for example angiogenesis. Such a modulator may be, for example an antagonist of a positive regulator of angiogenesis. Preferred modulators include antagonists of vascular endothelial growth factor (VEGF).

The use of one or more such modulators would be an optional feature to supplement the barrier function. The modulator or modulators may be adhered to the device, or may be provided directly on the mesh or material of the device. Alternatively, modulator or modulators may be placed in close proximity to the device, for example at or near to the site of implantation. The modulator or modulators may be introduced at the same time or subsequent to implantation of the device.

Such a modulator or modulators may be provided within a biodegradable matrix, such as collagen or gelatin. The matrix may be adhered to the surface of the device, or may be provided directly on the mesh or material of the device. Alternatively, the matrix comprising a modulator or modulators of an agent which regulates neo-vascularisation may be situated in close proximity to the to the device, for example at or near to the site of implantation.

A modulator of an agent which regulates neo-vascularisation, for example a modulator of an agent which regulates angiogenesis, is an agent that modulates the normal function of an agent which regulates neo-vascularisation, such that the effects of the agent which regulates neo-vascularisation are reduced (i.e., the modulator may be an antagonist) or increased (i.e., the modulator may be an agonist). The modulator may exert its effect through direct modulation, for example a modulator which binds to an agent which regulates angiogenesis itself, such that its activity is altered. Alternatively, the agent may exert its effect through indirect modulation, for example a modulator which binds to a receptor for an agent which regulates angiogenesis, such that transduction of signalling is altered. Any pharmaceutically acceptable modulator of an agent which regulates neo-vascularisation may be used in the present invention.

Preferred modulators for use in the present invention are modulators of positive regulators of angiogenesis, for example modulators of VEGF. Other preferred modulators are antagonists of an agent which regulates angiogenesis. More preferred modulators are antagonists of positive regulators of angiogenesis, for example antagonists of VEGF.

As used herein, the term antagonist of a positive regulator of angiogenesis means any agent which inhibits the activity of a positive regulator of angiogenesis polypeptide, inhibits the expression of that regulator (i.e. the process of transcription and translation of, for example, VEGF) or inhibits signalling of the regulator via a receptor for the regulator. Typically, an antagonist specific for a particular positive regulator of angiogenesis is used.

Any pharmaceutically acceptable modulator of an agent which regulates angiogenesis, for example a modulator, such as an antagonist, of a positive regulator of angiogenesis, can be used in the present invention. In particular, antagonists of vascular endothelial growth factor, for example vascular endothelial growth factor-A (VEGF-A) (Neufeld *et al*., 1994, Prog. Growth Factor Res. **5**, 89-97) or VEG-C (Ferrara, 1993, Trends Cardiovasc. Med. **3**, 244-250), basic fibroblast growth factor (bFGF) (Folkman and Shing, 1992, J. Biol. Chem. **267**, 10931-10934), hepatocyte growth factor (HGF) (Leung *et al*., 1989, Science **246**, 1306-1309), angiopoietin-1, insulin-like growth factor-1 and -2 (Tischer *et al*., 1989, Biochem. Biophys. Res. Commun. **165**, 1198-1206; Conn *et al*., 1990, Proc. Natl. Acad. Sc. USA **87**, 2628-2633)), epidermal growth factor (Maglione *et al:,* 1991, Proc. Natl. Acad. Sc. USA **88**, 9267-9271; DiSalvo *et al*., 1995, J. Biol. Chem. **270**, 7717-7723), platelet-derived growth factor (Tischer *et al*., 1991, J. Biol. Chem. 266, 11947-11954; Park et al., 1993, Mol. Biol. Cell **4**, 1317-1326) or sphingosine 1-phosphate (Maglione *et al*., 1993, Oncogene 8, 925-931) are useful in the invention. In addition, antagonists of the vasorelaxant prostacyclin may be useful in the invention. Typically antagonists of VEGF, preferably an antagonist of VEGF-A, are used in the invention.

Suitable VEGF antagonists include anti-VEGF antibodies and antigen-binding fragments thereof; receptor molecules which bind specifically to VEGF; agents which inhibit VEGF function, for example suramin and protein tyrosine kinase (PTK) inhibitors, for example lavendustin A (see for example, Waltenberger *et al*., 1996, J. Mol. Cell. Cardiol. **28**, 1523-1529; Hu *et al.,* 1995, Brit. J. Pharmacol. **114**, 262-268. VEGF antagonists also include agents which inhibit binding of VEGF to VEGF receptors or extracellular domains thereof, for example platelet factor 4 (PF-4) (see for example, Gengrinovitch *et al*., 1995, J. Biol. Chem **270**, 15059-15065). VEGF antagonists also include agents which inhibit VEGF receptor signalling; and agents which inhibit VEGF activation, for example mithramycin (see for example, Ryuto *et al*., 1996, J. Biol. Chem **271**, 28220-28228). VEGF anagtonists useful in the invention also include agents which are antagonists of signals that drive VEGF production, such as agents (for example drugs and other agents, including antibodies) which inhibit TGFβ or its ligands (see for example, Frank *et al*., 1995, J. Biol. Chem. **270**, 12607-12613; Pertovaara *et al*., 1994, J. Biol. Chem. **269**, 6271-6274). VEGF antagonists further include agents which are antagonists of signals taht drive VEGF production and/or synthesis.

An anti-VEGF antibody suitable for use in the invention is typically capable of inhibiting the activity of VEGF *in vivo*. Preferably the antibody will specifically bind to VEGF. An antibody, or other compound, specifically binds to VEGF, i.e. does not bind substantially to other proteins. A variety of protocols for competitive binding or immunoradiometric assays to determine the specific binding capability of an antibody are well known in the art Such immunoassays typically. involve the formation of complexes between the specific protein and its antibody and the measurement of complex formation.

The antibody may be polyclonal or monoclonal. The terms polyclonal and monoclonal refer to the degree of homogeneity of an antibody preparation and are not intended to be limited to particular methods of production.

Anti-VEGF antibodies for use in the invention may be antibodies to full length human VEGF polypeptides or fragments thereof. For the purposes of this invention, the term "antibody", unless specified to the contrary, includes fragments of whole antibodies which maintain their binding activity for a polypeptide encoded by a polynucleotide of the invention, a polypeptide of the invention or a fragment thereof. Such fragments include Fv, F(ab') and F(ab')₂ fragments, as well as single chain antibodies. Furthermore, the antibodies and fragment thereof may be chimeric antibodies, CDR-grafted antibodies or humanised antibodies.

Anti-VEGF antibodies can be produced by any suitable method. Means for preparing and characterising antibodies are well known in the art, see for example Harlow and Lane (1988) "Antibodies: A Laboratory Manual", Cold Spring Harbor Laboratory Press, Cold Spring Harbor, NY. For example, an antibody may be produced by raising antibody in a host animal against the whole polypeptide or a fragment thereof, for example an antigenic epitope thereof, herein after the "immunogen".

A method for producing a polyclonal antibody comprises immunising a suitable host animal, for example an experimental animal, with the immunogen and isolating immunoglobulins from the serum. The animal may therefore be inoculated with the immunogen, blood subsequently removed from the animal and the IgG fraction purified.

A method for producing a monoclonal antibody comprises immortalising cells which produce the desired antibody. Hybridoma cells may be produced by fusing spleen cells from an inoculated experimental animal with tumour cells (Kohler and Milstein (1975) *Nature* **256**, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by *in vitro* immunisation of human lymphocytes, followed by transformation of the lymphocytes with Epstein-Barr virus.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

Humanized antibodies may be obtained by replacing components of a non-human antibody with human components, without substantially interfering with the ability of the antibody to bind antigen.

Preferably, anti-VEGF antibodies for use in the invention (and antigen binding fragments thereof) are characterised by high affinity binding to VEGF, for example, high affinity binding to VEGF₁₂₁, VEGF₁₆₅, VEGF₁₈₉ or VEGF₂₀₆ and low toxicity (including HAMA and/or HACA response). An antibody where the individual components, such as the variable region, constant region and framework, individually and/or collectively possess low immunogenicity is particularly useful.

Examples of anti-VEGF antibodies are well known in the art, see for example Asano *et al*., 1995, Hybridoma **14**,475-480 and Kim *et al*., 1992, Growth Factors **7,** 53-64.

VEGF receptor molecules for use in the present invention bind specifically to VEGF and possess low immunogenicity. Preferably, the VEGF receptor molecule is characterised by high affinity binding to VEGF. VEGF receptor molecules include VEGF receptors, such as tyrosine kinase receptors, KDR, Flk, for example Flk-1, and Flt, for example Flt-1 and Flt-4. See for example, Lee *et al*., 1996, Proc. Natl. Acad. Sc. USA **93**, 1988-1992; deVries *et al*., 1992, Science **255**, 989-991; Quinn *et al*., Proc. Natl. Acad. Sc. USA **90**, 7533-7537; Shibuya *et al*., 1990, Oncogene **5**, 519-524; and Terman *et al*., 1992, Biochem. Biophys. Res. Commun. **187**, 1579-1586.

VEGF receptor molecules also include VEGF receptor multimeric molecules and VEGF immunoreceptor fusion molecules and derivatives and fragments thereof. VEGF receptor multimeric molecules can comprise all or a functional fragment of two or more VEGF receptors linked via one or more linkers. VEGF receptor multimeric molecules can further comprise a signal peptide of a secreted protein to direct expression of the multimeric molecule.

VEGF immunoreceptor fusion molecules can comprise at least one fragment of one or more immunoglobulin molecules and all or a functional fragment of one or more VEGF receptor(s). VEGF immunoreceptor fusion molecules can be assembled as monomers, or hetero- or homo- multimers. VEGF immunoreceptor fusion molecules can also be monovalent or multivalent. Examples of VEGF immunoreceptor fusion molecules are described by Aiello *et al*., Proc. Natl. Acad. Sci. USA **92**,10457-10461. See also Aiello *et al*., 1994 N. Engl. J. Med. **331**, 1480-1487; Park *et al*., J. Biol. Chem. **269**, 25646-25654.

A functional fragment or derivative of a VEGF receptor molecule refers to a fragment of the VEGF receptor polypeptide or a fragment of the VEGF receptor polynucleotide sequence that encodes the VEGF polypeptide, that is of sufficient size and sequence to functionally resemble VEGF receptor molecules that can be used in the present invention (for example which bind specifically to VEGF and possess low immunogenicity). A functional equivalent of a VEGF receptor molecule also includes modified VEGF receptor molecules. For example, a functional equivalent of VEGF receptor molecule can contain a "SILENT" codon or one or more amino acid substitutions, deletions or additions. For example, a functional equivalent of VEGF receptor molecule can contain a substitution of one or more codon encoding the same or different hydrophobic amino acid for another coding encoding a hydrophobic amino acid. See Ausubel *et al*., Current Protocols in Molecular Biology, Greene Publishing Assoc. and Wiley-Interscience, New York (1989).

VEGF antagonists suitable for use in the invention may also be oligonucleotides and modified oligonucleotides or peptides, for example oligonucleotides as disclosed in EP-A-978561 or peptide as disclosed in WO-A-9929861.

VEGF antagonists suitable for use in the invention may also be small molecules. Example small molecules include bicyclic heterocylic compounds as disclosed in WO-A-99/61444, mercapto-acylamino acid derivatives as disclosed in JP-A-10324625, quinoline and quinazoline derivates as disclosed in WO-A-0043366 and N-aryl(thio)anthracilic acid amide derivatives as disclosed in WO-A-0027820.

Any suitable assay can be carried out to identify an antagonist of VEGF, or indeed an antagonist of any agent which regulates angiogenesis. For example, assays may be carried out to determine whether a test substance is capable of antagonising expression and/or translation of VEGF. Such assays may be carried out using cell based systems, or using cell free recombinant systems. Alternatively, a cell can be stimulated using VEGF in the presence of a test substance and the amount of NO release measured, for example by use of the Griess reagent Reduced NO production as compared to a similar assay carried out in the absence of NO is indicative of the test substance being an antagonist of VEGF.

Suitable test substances for use in assays for identifying antagonists of VEGF include combinatorial libraries, defined chemical entities, peptides and peptide mimetics, oligonucleotides and natural product libraries. The test substances may be used in an initial screen of, for example, ten substances per reaction, and the substances of batches which show inhibition tested individually. Furthermore, antibody products (for example, monoclonal and polyclonal antibodies, single chain antibodies, chimaeric antibodies and CDR-grafted antibodies) may be used.

Preferred antagonists are those which antagonise an agent which regulates angiogenesis, for example VEGF, by at least 10%, at least 20%, at least 30%, at least 40%, at least 50%, at least 60%, at least 70%, at least 80%, at least 90%, at least 95% or at least 99% at a concentration of the inhibitor of 1 µgml⁻¹, 10 µgml⁻¹, 100 µgml⁻¹, 500 µgml⁻¹, 1 mgml⁻¹, 10 mgml⁻¹, 100mg ml⁻¹ as determined in an assay as described above. Preferably, the antagonist will achieve substantially total antagonism, that is preferably it will substantially completely inhibit activity in the assay.

The percentage antagonism represents the percentage decrease in "activity", however that is measured, in a comparison of assays carried out in the presence and absence of the test substance. Any combination of the above mentioned degrees of percentage inhibition and concentration of inhibitor may be used to define an antagonist for use in the invention, with greater antagonism at lower concentrations being preferred.

According to the preferred use of the invention, the device is implanted at the time of primary varicose vein surgery to reduce the number of surgical procedures and to avoid the more difficult surgery for recurrent varicose veins.

## Claims

1. A medical device comprising first (20) and second (22) lamellar portions joined by a connecting bridge (24), wherein said device is constructed such that said first and second portions are capable of being positioned on opposite sides of the cribriform fascia (10), with said connecting bridge passing therethrough, and wherein said first and second portions are barriers to neo-vascularisation.

2. A device according to claim 1, wherein the first and second lamellar portions are substantially rectangular, or ovoid.

3. A device according to claim 2, wherein the short sides of the rectangular first lamellar portion are substantially parallel to the long sides of the second lamellar portion.

4. A device according to claim 1, 2 or 3, wherein the first lamellar portion comprises a flap which is locatable around at least a portion of the periphery of the deep femoral vein (2).

5. A device according to any one of the preceding claims, wherein the connecting bridge is approximately cylindrical in shape.

6. A device according to any one of the preceding claims which is soft and flexible.

7. A device according to any one of the preceding claims made from at least one of PTFE, Dacron™, polypropylene, and Marlex™ or other similar implantable, non-biodegradable material.

8. A device according to any one of the preceding claims wherein said first and second lamellar portions and said connecting bridge are all made of the same material.

9. A device according to any one of the preceding claims made of a mesh-like material.

10. A device according to claim 9, wherein the pore size of the mesh substantially prevents the growth of blood vessels therethrough.

11. A device according to any one of the preceding claims which is wholly implantable within a patient.

12. A device according to claim 11, which can be sutured into place.

13. A device according to any one of the preceding claims coated with at least one modulator of an agent which regulates neo-vascularisation.

14. A device according to claim 13, wherein a said modulator of an agent which regulates neo-vascularisation comprises an antagonist of vascular endothelial growth factor.

15. A device according to claim 13 or 14, wherein said at least one agent is provided within a biodegradable matrix.

16. A device according to claim 15, wherein said biodegradable matrix comprises at least one of collagen and gelatin

## Patentansprüche

1. Medizinische Vorrichtung, umfassend erste (20) und zweite (22) lamellare Bereiche, die mit einer Verbindungsbrücke (24) verbunden sind, wobei die Vorrichtung so konstruiert ist, dass die ersten und zweiten Bereiche auf gegenüberliegenden Seiten der Fascia cribrosa (10) positioniert werden können, wobei die Verbindungsbrücke durch diese hindurchgeht und wobei die ersten und zweiten Bereiche Barrieren gegen Neovaskularisation darstellen.

2. Vorrichtung nach Anspruch 1, wobei die ersten und zweiten lamellaren Bereiche im wesentlichen rechteckig oder ovoid sind.

3. Vorrichtung nach Anspruch 2, wobei die kurzen Seiten des rechteckigen ersten lamellaren Bereiches im wesentlichen parallel zu den langen Seiten des zweiten lamellaren Bereiches verlaufen.

4. Vorrichtung nach Anspruch 1, 2 oder 3, wobei der erste lamellare Bereich eine Klappe umfasst, die um mindestens einen Bereich des Umfangs der Vena profunda femoris (2) herum anbringbar ist.

5. Vorrichtung nach einem der vorstehenden Ansprüche, wobei die Verbindungsbrücke eine annähernd zylindrische Gestalt aufweist.

6. Vorrichtung nach einem der vorstehenden Ansprüche, die weich und flexibel ist.

7. Vorrichtung nach einem der vorstehenden Ansprüche, hergestellt aus mindestens einem der Materialien PTFE, Dacron^{R}, Polypropylen und Marlex^{R} oder einem ähnlichen, implantierbaren, biologisch nichtabbaubaren Material.

8. Vorrichtung nach einem der vorstehenden Ansprüche, wobei die ersten und zweiten lamellaren Bereiche und die Verbindungsbrücke alle aus dem gleichen Material gefertigt sind.

9. Vorrichtung nach einem der vorstehenden Ansprüche, hergestellt aus einem maschenartigen Material.

10. Vorrichtung nach Anspruch 9, wobei die Porengröße der Maschen im wesentlichen das Wachstum von durchtretenden Blutgefäßen verhindert.

11. Vorrichtung nach einem der vorstehenden Ansprüche, die einem Patienten vollständig implantierbar ist.

12. Vorrichtung nach Anspruch 11, die festgenäht werden kann.

13. Vorrichtung nach einem der vorstehenden Ansprüche, die mit mindestens einem Modulator eines Mittels, das die Neovaskularisation reguliert, beschichtet ist.

14. Vorrichtung nach Anspruch 13, wobei der Modulator eines Mittels, das die Neovaskularisation reguliert, einen Antagonisten des vaskulären endothelialen Wachstumfaktors umfasst.

15. Vorrichtung nach Anspruch 13 oder 14, wobei das mindestens eine Mittel innerhalb einer biologisch abbaubaren Matrix bereitgestellt wird.

16. Vorrichtung nach Anspruch 15, wobei die biologisch abbaubare Matrix mindestens einen der Bestandteile Kollagen und Gelatine umfasst.

## Revendications

1. Dispositif médical comprenant une première (20) et une seconde (22) portions lamellaires réunies par un pont de jonction (24), ledit dispositif étant construit de telle façon que lesdites première et seconde portions soient aptes à être positionnées de part et d'autre de la fascia cribriformis (10), ledit pont de jonction traversant celle-ci, et lesdites première et seconde portions étant des barrières à la néo-vascularisation.

2. Dispositif selon la revendication 1, dans lequel lesdites première et seconde portions lamellaires sont substantiellement rectangulaires ou ovoïdes.

3. Dispositif selon la revendication 2, dans lequel les petits côtés de la première portion lamellaire rectangulaire sont substantiellement parallèles aux grands côtés de la seconde portion lamellaire.

4. Dispositif selon la revendication 1, 2 ou 3, dans lequel la première portion lamellaire comprend une partie enveloppante qui peut être positionnée autour d'au moins une partie de la périphérie de la veine fémorale profonde (2).

5. Dispositif selon l'une quelconque des revendications précédentes, dans lequel le pont de jonction a une forme approximativement cylindrique.

6. Dispositif selon l'une quelconque des revendications précédentes, qui est souple et flexible.

7. Dispositif selon l'une quelconque des revendications précédentes, fabriqué en au moins un parmi le PTFE, le Dacron™, le polypropylène et le Marlex™ ou d'autres matériaux similaires, implantables, non-biodégradables.

8. Dispositif selon l'une quelconque des revendications précédentes, dans lequel lesdites première et seconde portions lamellaires et ledit pont de jonction sont tous faits du même matériau.

9. Dispositif selon l'une quelconque des revendications précédentes, fabriqué en un matériau du type filet.

10. Dispositif selon la revendication 9, dans lequel la dimension des pores du filet empêche substantiellement la croissance de vaisseaux sanguins au travers de celui-ci.

11. Dispositif selon l'une quelconque des revendications précédentes, qui est totalement implantable chez un patient.

12. Dispositif selon la revendication 11, qui peut être maintenu en place par une suture.

13. Dispositif selon l'une quelconque des revendications précédentes, enduit d'au moins un modulateur d'un agent qui régule la néo-vascularisation.

14. Dispositif selon la revendication 13, dans lequel ledit modulateur d'un agent qui régule la néo-vascularisation comprend un antagoniste d'un facteur de croissance endothéliale vasculaire.

15. Dispositif selon la revendication 13 ou 14, dans lequel ledit au moins un agent est dans une matrice biodégradable.

16. Dispositif selon la revendication 15, dans lequel ladite matrice biodégradable comprend au moins un parmi le collagène et la gélatine.
